# EUROPEAN PATENT APPLICATION

(11) **EP 4 644 520 A1**
(43) Date of publication of application: **05.11.2025**
(21) Application number: 23912425.8
(22) Date of filing: 27.07.2023
(51) Int. Cl.: C12M 1/00, C12M 3/00, C12M 1/34

(54) **VERTICAL CULTURE SLIDE DEVICE FOR TISSUE OR CELL CULTURE**

(30) Priority: 29.12.2022 KR 20220189919
(71) Applicant: Daegu Gyeongbuk Institute of Science and Technology, Daegu 42988 (KR)
(72) Inventor: LEE, Chang-Hun, Seoul 06973 (KR); LEE, Gyeonghee, Yangsan-si Gyeongsangnam-do 50572 (KR); LEE, Dong Gyu, Gwangju 62356 (KR); HAN, Min Ae, Daegu 43008 (KR)
(74) Representative: Roos, Peter
(86) International application number: PCT/KR2023/010957
(87) International publication number: WO 2024/143738

(57) **Abstract**

The present invention provides an upright culture slide device for culturing tissues or cells by which a tissue development process, a drug response, a structural or physical change due to genetic mutation, and so on may be easily studied by forming a culture space where cells or tissues are cultured between a pair of slides having opposing surfaces facing each other with a corresponding gap, by providing a culture environment where cell division, growth, and differentiation patterns may be observed and recorded in real-time while culturing various tissues and organs having a layered structure, and by observing and measuring in real time the structural and physiological characteristics that appear during a culture process of cells, living tissues, organoids, artificial skin, and other biological cultures that form stratified layers.

## Description

### Technical Field

The present invention relates to an upright culture slide device for tissue or cell culture, and more specifically, to an upright culture slide device for tissue or cell culture that enables real-time observation of a structure of a culture body and measurement of physical properties or structural robustness, and so on during a process of culturing cells, tissues, organoids, and other biological derivatives.

### Background Art

Cell culture is an important technique in biological research including molecular biology, and refers to culturing specific cells to diagnose or treat human diseases.

Cell culture is generally performed by using a cell culture device, and the conventional cell culture device includes a culture dish and a cover, and transparent materials (glass and so on), such as cover glass or slide glass, are added thereto when necessary.

A method for culturing cells and observing them using a conventional cell culture device is as follows.

Cells treated with drugs or cells recombined with specific genes and a culture solution are placed on a thin cover glass in a culture dish, covered with a lid, and then the covered culture dish is placed in an incubator, and accordingly, the cell culture preparation is completed.

The incubator provides a specific environment suitable for cell culture, allowing cells to proliferate. Once cell culture is complete, the cover glass with cells is placed on a slide glass with a tweezer, and the cells are observed through a microscope.

However, since cells proliferate by spreading out in a width direction on a two-dimensional plane, in such a conventional method, the upper and lower surfaces of cells or tissues can be observed, but the side surfaces thereof cannot be observed. In order to observe the side surfaces of cells or tissues, cell culture has to be stopped, and the side surfaces have to be observed after the cell fixation process; that is, the cells are killed.

Also, such a conventional method requires a process, such as fixation and sectioning (side cutting), which makes it difficult to observe live cells in real time.

A cell culture device having a structure in which two glass slides overlap each other has been developed to culture cells while observing side surfaces of cells or tissues. However, such a cell culture device has inconvenient methods for separating chambers from each other, fixing a glass slide to a chamber, and separating the fixed chamber from the glass slide. There is a problem that the glass slide is easily broken during a process of separating the chamber from the glass slide. Additionally, this method does not allow for easy replacement of the culture solution, and it is impossible to form an air-liquid interface on top of the cells.

Conventional technology includes Korean Patent No. 10-1215657 (2012.12.18).

Also, 2.5D culture or 3D culture, which allows cells or tissues to grow and differentiate in a three-dimensional structure environment by using a 3D bioprinter or well insert, is actively being used, breaking away from the 2D culture in which cells are cultured on a flat plate as in the past.

The development of microfluidic culture methods for studying trace samples, such as organ-on-a-chip, is also increasing.

However, all newly emerging culture methods essentially spread cultured bodies, such as cells, on a two-dimensional XY plane, and there is a problem that, although an upper structure may be observed under a microscope during the culture process, a side structure may be difficult to observe under a microscope.

### Disclosure of Invention

### Technical Problem

An objective of the present invention is to provide an upright culture slide device for culturing tissues or cells by which a tissue development process, a response to drug, a structural or physical change due to genetic mutation, and so on may be easily studied by providing a culture environment where cell division, growth, and differentiation patterns may be observed and recorded in real-time while culturing various tissues and organs having a layered structure, and by observing and measuring in real time the structural and physiological characteristics that appear during a culture process of cells, living tissues, organoids, artificial skin, and other biological cultures that form stratified layers.

### Solution to Problem

An upright culture slide device for tissue or cell culture according to the present invention includes a pair of slides having opposing surfaces facing each other and arranged to be separated from each other, a culture solution-accommodating gasket provided between the pair of slides and having an open upper portion to form a culture solution chamber for accommodating a culture solution, a cell-accommodating gasket provided inside the culture solution-accommodating gasket, including a wall of a culture space in which cells or tissues are cultured, having an open upper portion, and including a support that supports the culture space, and a semipermeable membrane support layer formed in the cell-accommodating gasket and allowing a culture solution accommodated in the culture solution-accommodating gasket to flow into the cell-accommodating gasket.

In this case, according to the present invention, the cell-accommodating gasket includes a horizontal bar having a length in a horizontal direction, and vertical bars formed respectively on the left and right sides of the horizontal bar to have lengths in a vertical direction and having upper sides forming the wall of the culture space, and having lower sides forming the support.

Here, according to the present invention, the lower sides of the vertical bars of the cell-accommodating gasket which form the horizontal bar and the support have rough surfaces such that the culture solution may move along the rough surfaces.

In addition, the upright culture slide device for tissue or cell culture according to the present invention further includes a cover coupled to the upper sides of the pair of slides facing each other and sealing upper portions of the culture space and the culture solution chamber from the outside, and a stand coupled to lower sides of the pair of slides facing each other and causing the pair of slides to upright in a vertical direction.

Also, the upright culture slide device for tissue or cell culture according to the present invention includes, a plurality of culture solution-accommodating gaskets and a plurality of cell solution-accommodating gaskets, which are provided between the pair of slides facing each other to form a plurality of culture solution chambers and the culture space.

An upright culture slide device for tissue or cell culture according to the present invention includes a pair of slides having opposing surfaces facing each other with a corresponding interval, and a spacer member provided between the pair of slides, forming a culture space, in which cells or tissues are cultured, in a center of the spacer member, and forming a culture solution chamber, in which a culture solution is accommodated, around the culture space.

In this case, according to the present invention, the spacer member forms a space having a corresponding area, and includes a culture solution chamber and a culture space partitioned by a partition wall provided in the space and forming an outer wall of the culture space.

In addition, according to the present invention, an upper side of the partition wall of the spacer member has a culture solution inlet/outlet hole through which a culture solution is injected into the culture solution chamber from an outside or the culture solution accommodated in the culture solution chamber is discharged to the outside, and an air inlet/outlet hole through which air in the culture solution chamber flows to the outside when the culture solution is injected into the culture solution chamber.

Also, according to the present invention, a microchannel, through which the culture solution is supplied to the culture space to the culture chamber due to a capillary phenomenon, is formed on a lower side of the partition wall forming the outer wall of the culture space.

In addition, according to the present invention, the bottom surface of the culture space formed by the partition wall is formed as an inclined surface corresponding to the flow direction of the culture solution when the culture solution is injected into the culture solution chamber.

Also, according to the present invention, a semipermeable membrane support layer is formed on the lower side of the culture space to support cells or tissues cultured within the culture space.

Here, according to the present invention, a fixed hole is formed by extending a part of the lower side of the semipermeable membrane support layer on the lower side of the partition wall that forms the outer wall of the culture space.

In addition, according to the present invention, the upright culture slide device for tissue or cell culture further includes a pair of trans-epithelial electrical resistance (TEER) measuring electrodes, one of which is installed in the culture space and the other of which is installed in the culture solution chamber, measuring electrical resistance between the culture space and the culture solution chamber.

Here, according to the present invention, the trans-epithelial electrical resistance measuring electrode enters the culture space and the culture solution chamber by being guided by an electrode guide hole formed in an extended portion that is formed by extending an inner surface of the partition wall forming the outer wall of the culture space by a corresponding length, and by an electrode guide hole formed adjacent to the culture solution inlet/outlet hole formed on the upper side of the partition wall.

In addition, according to the present invention, the spacer member includes a first spacer pad forming a space having a corresponding area therein and including a culture solution chamber and a culture space formed in the space and partitioned by a first partition wall forming the outer wall of the culture space, and a second spacer pad forming a space having a corresponding area therein and including a culture solution chamber and a culture space formed in the space and partitioned by a second wall forming the outer wall of the culture space, and the first spacer pad is coupled to the second spacer pad.

### Advantageous Effects of Invention

An upright culture slide device for tissue or cell culture according to the present invention has the following effects.

Since a culture cartridge forms a sandwich structure by using two transparent outer wall members, real-time microscopic observation is possible. Also, recording through video and/or time-lapse photography are possible.

In particular, the conventional flat-panel structure has an advantage in that a side surface (a side surface of a cell-layered structure, an internal structure of tissue) which may only be observed by tearing, fixing, and cutting a culture body may be photographed in real time during a culture process without killing the tissue.

Also, there is an advantage of minimizing the growth time in a width direction in stratified tissue, which is formed by the differentiation of multiple layers of cells such as skin.

In addition to skin, it may be used for various tissues (for example, a respiratory alveolar structure, an epidermal structure of the bladder, a surface structure of digestive system intestinal tissues, differentiation of a cortex-endothelium structure of the brain, eyeballs, and so on) that are divided and differentiated in a layered structure.

In addition, the present invention may be used for real-time observation of organoids having various internal and external structural forms, and in a case of a device including electrodes installed above and below a culture space where cells are cultured, a structural strength or moisture content (or loss amount) of cultured tissue may be measured in real time during a culture process by measuring trans-epithelial electrical resistance (TEER).

### Brief Description of Drawings

FIG. 1 illustrates example views of a configuration of an upright culture slide device for tissue or cell culture according to a first embodiment of the present invention.
FIG. 2 illustrates example views of a state in which cells and tissues are cultured in a culture space of the upright culture slide device for tissue or cell culture according to the first embodiment of the present invention.
FIG. 3 illustrates example views of a state in which a plurality of culture spaces and culture solution chambers according to the first embodiment of the present invention are formed.
FIG. 4 illustrates example views of a state in which a cover and a stand are coupled to the upright culture slide device for tissue or cell culture according to the first embodiment of the present invention.
FIG. 5 illustrates example views of a state in which a stand is coupled to the upright culture slide device for tissue or cell culture according to the first embodiment of the present invention in place of a clamp.
FIG. 6 illustrates example views of a configuration of an upright culture slide device for tissue or cell culture according to a second embodiment of the present invention.
FIG. 7 is an example view illustrating a state in which a culture solution is injected into the upright culture slide device for tissue or cell culture according to the second embodiment of the present invention.
FIG. 8 illustrates example views of a state in which a cover is coupled to the upright culture slide device for tissue or cell culture according to the second embodiment of the present invention.
FIG. 9 illustrates example views of a state in which a TEER electrode is included in the upright culture slide device for tissue or cell culture according to the second embodiment of the present invention.
FIG. 10 illustrates example views of a state in which a plurality of upright culture slide devices for tissue or cell culture according to the second embodiment of the present invention are accommodated in a culture box.

### Best Mode for Carrying out the Invention

The present invention provides an upright culture slide device for tissue or cell culture including a pair of slides having opposing surfaces facing each other and arranged to be separated from each other, a culture solution-accommodating gasket provided between the pair of slides and having an open upper portion to form a culture solution chamber for accommodating a culture solution, a cell-accommodating gasket provided inside the culture solution-accommodating gasket, including a wall of a culture space in which cells or tissues are cultured, having an open upper portion, and including a support that supports the culture space, and a semipermeable membrane support layer formed in the cell-accommodating gasket and allowing a culture solution accommodated in the culture solution-accommodating gasket to flow into the cell-accommodating gasket.

### Mode for the Invention

Hereinafter, preferred embodiments of the present invention will be described in detail with reference to the attached drawings. Prior to this, terms or words used in the present specification and claims should not be interpreted as limited to their usual or dictionary meanings, and should be interpreted as meanings and concepts that conform to the technical idea of the present invention based on the principle that at least one inventor may appropriately define concepts of terms to describe his or her own invention in the best way.

Therefore, the embodiments described in the present specification and the configurations illustrated in the drawings are merely the most preferred embodiments of the present invention, and do not represent all of the technical ideas of the present invention, and accordingly, it should be understood that there may be equivalent modification examples that may be replaced at the time of the present application.

The present invention relates to an upright culture slide device for culturing tissues or cells, which provides a an upright culture slide device for culturing tissues or cells by which a tissue development process, a drug response, a structural or physical change due to genetic mutation, and so on may be easily studied by providing a culture environment where cell division, growth, and differentiation patterns may be observed and recorded in real time while culturing various tissues and organs having a layered structure, and by observing and measuring in real time the structural and physiological characteristics that appear during a culture process of cells, living tissues, organoids, artificial skin, and other biological cultures that form stratified layers, and will be described below with reference to the drawings.

An upright culture slide device for culturing tissues or cells according to an embodiment of the present invention is stored in a vertically upright state, and forms a culture space therein to receive a culture solution due to a capillary phenomenon and to culture cells.

In this case, the cells or tissues cultured in the upright culture slide device for culturing the tissues or cells may include all or part of epidermis, dermis, subdermis, full-thickness skin tissue model, hair root, hair, and secretory glands derived from the skin, and the cultured cells may include major cells (keratinocyte, fibroblast, melanocyte, and so on) of the skin but are not limited to the types described above.

Similarly, the cultured cells may include major tissues and derived cells of a human body and animal, including the integumentary system, nervous system, endocrine system, respiratory system, circulatory system, digestive system, excretory system, and reproductive system but are not limited to the types described above.

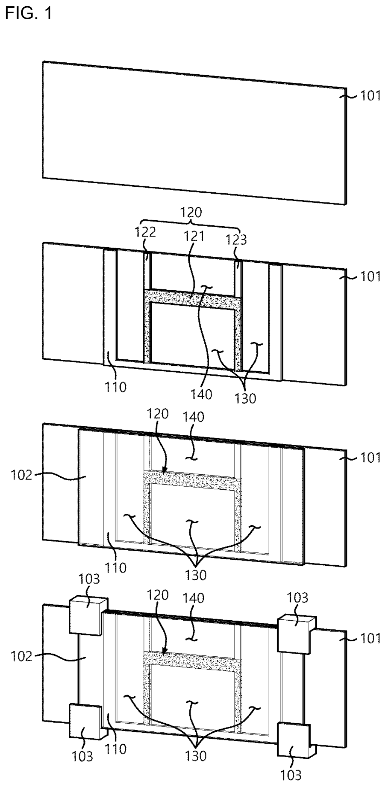

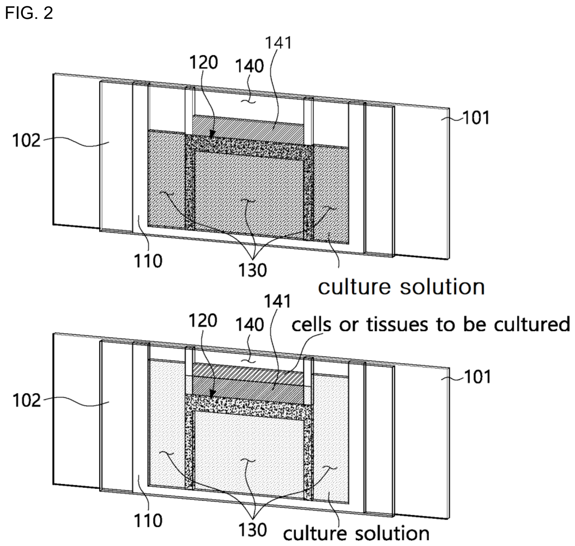

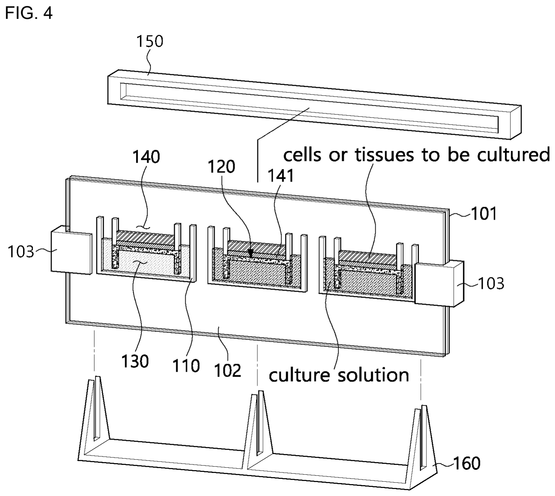

An upright culture slide device for tissue or cell culture according to a first embodiment of the present invention described with reference to FIGS. 1 to 5 includes a pair of slides 101 and 102, a culture solution-accommodating gasket 110, and a cell-accommodating gasket 120, wherein the pair of slides 101 and 102 are provided with opposing surfaces facing each other with a corresponding gap.

In this case, the pair of slides 101 and 102 may be made of a transparent acrylic plate or glass plate, or polymer (PMMA) such that the inside may be seen through the plate shape but is not limited thereto, and any transparent material may be used.

In addition, the culture solution-accommodating gasket 110 forms a ' 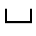 ' shape and is provided between the pair of slides 101 and 102 to form an outer wall of a culture solution chamber 130 which is open at the top and accommodates a culture solution in the inside.

Therefore, a gap between the pair of slides 101 and 102 may be determined by a thickness of the culture solution-accommodating gasket 110, and a clamp 103 such as a pair of forceps for fixing the pair of slides 101 and 102 facing each other may be placed on outer peripheries (corners) of the slides 101 and 102 to fix the pair of slides 101 and 102 facing each other.

Here, a material of the culture solution-accommodating gasket 110 is preferably formed of elastic rubber, silicone, foamed elastic material, or so on such that a lower portion, a left portion, and a right portion of the culture solution chamber 130 may be sealed between a pair of slides 101 and 102.

Also, the cell-accommodating gasket 120 is provided inside the culture solution-accommodating gasket 110, and the cell-accommodating gasket 120 is provided inside the culture solution-accommodating gasket 110 and includes a wall of the culture space 140 in which cells or tissues are cultured, has an open upper portion, and includes a support that supports the culture space 140.

Therefore, the culture solution chamber 130 is formed, by the culture solution-accommodating gasket 110, between a pair of slides 101 and 102 with opposing surfaces facing each other at a corresponding interval, and the cell-accommodating gasket 120 is provided inside the culture solution-accommodating gasket 110 to form the culture space 140.

Here, the cell-accommodating gasket 120 has a single horizontal bar 121 in the center, and a pair of vertical bars 122 and 123 are connected parallel to each other on the left and right sides of the horizontal bar 121 to forming an 'H' shape.

In this case, it is preferable that the horizontal bar 121 is located at the center of the length of the vertical bars 122 and 123, and upper sides of the vertical bars 122 and 123 form the left and right walls of the culture space 140, in which cells or tissues are cultured, with respect to the horizontal bar 121, and lower sides of the vertical bars 122 and 123 forms a support that supports the culture space 140 with respect to the horizontal bar 121.

Here, a lower side of the horizontal bar 121 of the cell-accommodating gasket 120 and the lower sides of the vertical bars 122 and 123 are roughened, and accordingly, a culture solution accommodated in the culture solution chamber 130 may move to the culture space 140 along the roughened rough surface.

Therefore, the culture space 140 formed by the cell-accommodating gasket 120 may receive the culture solution accommodated in the culture solution chamber 130 formed by the culture solution-accommodating gasket 110 due to a capillary phenomenon through the horizontal bar 121 having the roughened surface, and accordingly, cells or tissues are cultured.

Also, a semipermeable membrane support layer 141 is formed on a lower side of the culture space 140 of the cell-accommodating gasket 120, and the semipermeable membrane support layer 141 may support cells or tissues cultured in the culture space 140.

The semipermeable membrane support layer 141 is formed of one of a semipermeable membrane, an extracellular matrix, a hydrogel, or a combination thereof, and may prevent the cells cultured in the culture space 140 from falling, while allowing selective material exchange with a culture solution or providing only signal materials that promote cell division and cell differentiation in the culture solution.

In this case, a semipermeable membrane may be formed of a scaffold material which is one of PES, PS, PP, PET, cellulose, nitrocellulose, and cellulose acetate, or a combination thereof, and the extracellular matrix may be formed of one of collagen, gelatin, hyaluronic acid, and Matrigel, or a combination thereof, and the hydrogel may be formed of any one of collagen, gelatin, hyaluronic acid, Matrigel, GeIMA, CoIMA, fibrin, cellulose, hemicellulose, agarose, and alginic acid, or a combination thereof.

The extracellular matrix or hydrogel may include cross-linking to provide physical properties, and a methacrylated material or metal ions (for example, magnesium ions, calcium ions, and so on) may be used for the cross-linking, and UV curing or thermal curing may be used for a cross-linking reaction.

In addition, an upper side of the culture space 140 may be exposed to the atmosphere to form an air-liquid interface, and a part of the culture space 140 may exclude a semipermeable membrane, an extracellular matrix, or hydrogel, allowing only air and culture solution to pass therethrough.

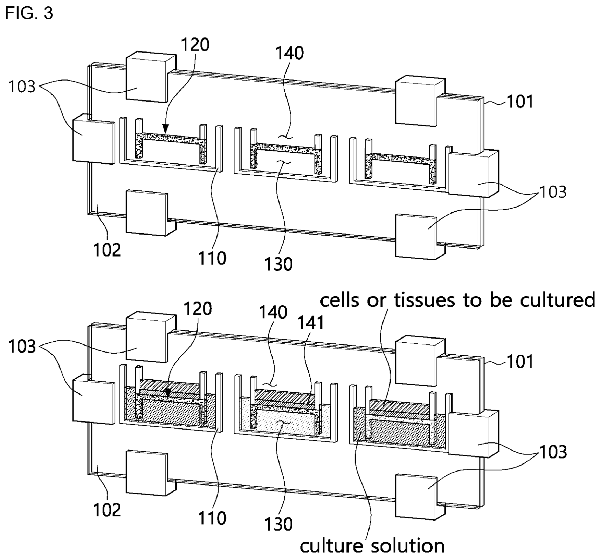

In addition, referring to FIG. 3, the upright culture slide device for tissue or cell culture according to the first embodiment of the present invention may include a plurality of culture solution-accommodating gaskets 110 and a plurality of cell solution-accommodating gaskets 120 between the pair of slides 101 and 102 facing each other, and accordingly, a plurality of culture solution chambers 130 and a plurality of culture spaces 140 may be formed.

Also, referring to FIG. 3, the upright culture slide device for tissue or cell culture according to the first embodiment of the present invention may further include a cover 150 and a stand 160, and the cover 150 may be coupled to upper sides of the pair of slides 101 and 102 facing each other, and accordingly, the culture space 140 of the cell solution-accommodating gasket 120 and an upper portion of the culture solution chamber 130 of the culture solution-accommodating gasket 110 may be sealed.

In addition, the stand 160 is coupled to lower sides of the pair of slides 101 and 102 facing each other to upright vertically the pair of slides 101 and 102.

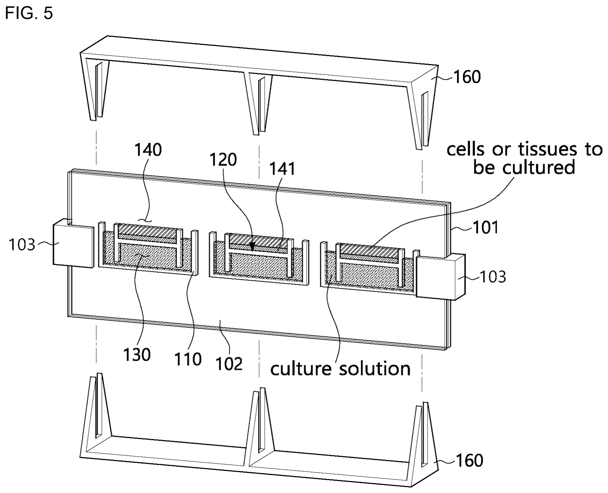

Referring to FIG. 5, the upright culture slide device for tissue or cell culture according to the first embodiment of the present invention may use the cover 150 and the stand 160 instead of the clamp 103 that fixes the pair of slides 101 and 102.

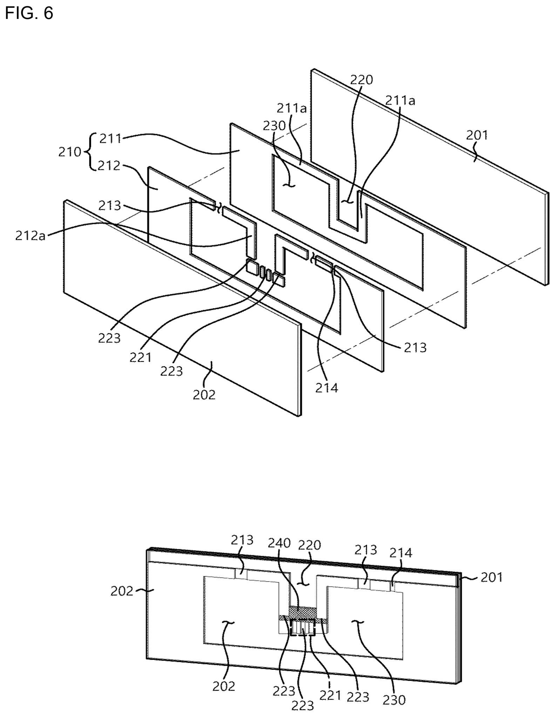

Referring to FIGS. 6 to 10, the upright culture slide device for tissue or cell culture according to the second embodiment of the present invention includes a pair of slides 201 and 202 and a spacer member 210, and first, opposing surfaces of the pair of slides 201 and 202 face each other with a corresponding gap.

In this case, the pair of slides 201 and 202 may each have a plate shape and be formed of a transparent acrylic plate, a transparent glass plate, or polymer (PMMA) such that insides of the pair of slides 201 and 202 may be seen but are not limited thereto, and any transparent material may be used.

In addition, the spacer member 210 is provided between the pair of slides 201 and 202 having opposing surfaces face each other with a corresponding interval, and the spacer member 210 has a central upper side communicating with the outside and forms a culture space 220 in which cells or tissues are cultured, and forms a culture solution chamber 230 in which a culture solution is accommodated around the culture space 220.

In this case, the spacer member 210 is formed by coupling a first spacer pad 211 having a plate shape to a second spacer pad 212 having a plate shape, the first spacer pad 211 has a rectangular space therein, and a first partition wall 211a having a shape of ' 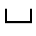 ' is formed in the space to divide the culture solution chamber 230 from the culture space 220.

In this case, the first partition wall 211a forms an outer wall of the culture space 220, and a central part thereof is connected to the outside and forms the culture space 220 where cells or tissues are cultured, and the culture solution chamber 230 accommodating a culture solution is formed around the culture space 220.

An upper side end of the first partition wall 211a is connected to an upper side of the first spacer pad 211 to close the culture solution chamber 230.

In addition, the second spacer pad 212 also forms a rectangular space therein, and a second partition wall 212a having a shape of ' 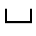 ' is formed in the space to divide the culture solution chamber 230 from the culture space 220.

In this case, the second partition wall 212a forms an outer wall of the culture space 220, and a central part thereof is connected to the outside and forms the culture space 220 where cells or tissues are cultured, and the culture chamber 230 accommodating a culture solution is formed around the culture space 220.

An upper end of the second partition wall 212a is connected to an upper side of the second spacer pad 212 to close the culture chamber 230.

Here, it is preferable to form at least two or more culture solution inlet/outlet holes 213 on an upper side of the second partition wall 212a such that a culture solution may be injected from the outside into the culture chamber 230 or a culture solution accommodated in the culture chamber 230 may be discharged to the outside, and to form at least one air inlet/outlet hole 214 on the upper side.

Inflow and outflow of a culture solution may be performed by using a syringe needle that may enter the culture solution inlet/outlet hole 213.

In addition, it is preferable to form a microchannel 221 on a lower side of the second partition wall 212a forming the bottom of the culture space 220 such that a culture solution may be supplied to the culture space 220 from the culture solution chamber 230 partitioned by the second partition wall 212a due to a capillary phenomenon.

Although the upright culture slide device for tissue or cell culture according to the second embodiment of the present invention is described as being limited to forming the culture solution inlet/outlet holes 213, the air inlet/outlet hole 214, and the microchannel 221 in the second partition wall 212a of the second spacer pad 212, the present invention not limited thereto, and the culture solution inlet/outlet holes 213, the air inlet/outlet hole 214, and the microchannel 221 may also be formed in the first partition wall 211a of the first spacer pad 211.

Therefore, the culture solution inlet/outlet holes 213, the air inlet/outlet hole 214, and the microchannel 221 may be formed in either the first partition wall 211a of the first spacer pad 211 or the second partition wall 212a of the second spacer pad 212, and may be formed in both the first partition wall 211a of the first spacer pad 211 and the second partition wall 212a of the second spacer pad 212, and the culture solution inlet/outlet holes 213, the air inlet/outlet hole 214, and the microchannel 221 may be formed in the first partition wall 211a of the first spacer pad 211 and the second partition wall 212a of the second spacer pad 212 to be dispersed.

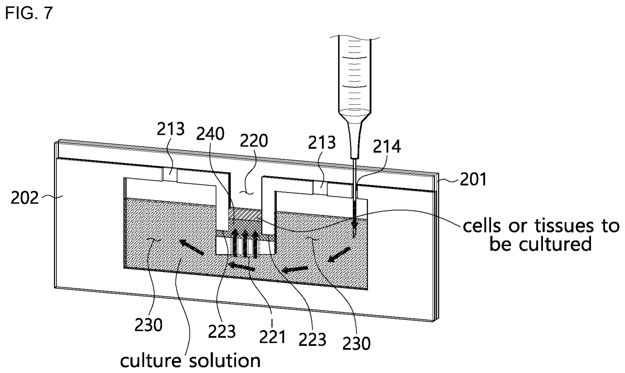

Also, referring to FIG. 7, in an upright culture slide device for tissue or cell culture according to a second embodiment of the present invention, a bottom surface of the culture space 220 forms an inclined surface 222 corresponding to a flow direction when a culture solution is injected to prevent bubbles, which are generated when a culture solution is injected into the culture solution chamber 230, from being generated.

A semipermeable membrane support layer 240 is formed at the bottom of the culture space 220 to be able to support cells or tissues cultured in the culture space 220.

The semipermeable membrane support layer 240 is formed of a semipermeable membrane, an extracellular matrix, a hydrogel, or a combination thereof, and may prevent the cells cultured in the culture space 220 from falling, while allowing selective material exchange with a culture solution or providing only signal materials that promote cell division and cell differentiation in the culture solution.

In this case, a semipermeable membrane may be formed of a scaffold material which is one of PES, PS, PP, PET, cellulose, nitrocellulose, and cellulose acetate, or a combination thereof, and the extracellular matrix may be formed of one of collagen, gelatin, hyaluronic acid, and Matrigel, or a combination thereof, and the hydrogel may be formed of any one of collagen, gelatin, hyaluronic acid, Matrigel, GeIMA, CoIMA, fibrin, cellulose, hemicellulose, agarose, and alginic acid, or a combination thereof.

The extracellular matrix or hydrogel may include cross-linking to provide physical properties, and a methacrylated material or metal ions (for example, magnesium ions, calcium ions, and so on) may be used for the cross-linking, and UV curing or thermal curing may be used for a cross-linking reaction.

In addition, an upper side of the culture space 220 may be exposed to the atmosphere to form an air-liquid interface, and a part of the culture space 220 may exclude a semipermeable membrane, an extracellular matrix, or hydrogel, allowing only air and culture solution to pass therethrough.

Here, a fixed hole 223 formed by extending a part of a lower side of the semipermeable membrane support layer 240 is provided on a lower side of the second partition wall 212a forming an outer wall of the culture space 220, and the semipermeable membrane support layer 240 is fixed to the culture space 220 by a part of the lower side of the semipermeable membrane support layer 240 formed in the fixed hole 223.

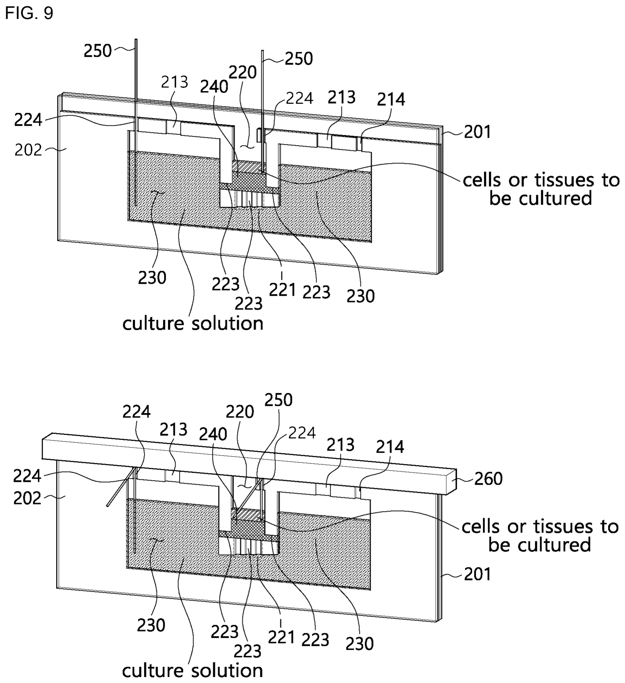

Also, referring to FIG. 9, the upright culture slide device for tissue or cell culture according to the second embodiment of the present invention may include a trans-epithelial electrical resistance (TEER) measuring electrode 250 that may measure electrical resistance between the culture space 220 and the culture chamber 230 so as to measure physical robustness or durability of cells or tissues being cultured in real time.

The trans-epithelial electrical resistance measuring electrode 250 is a pair, one of which is installed in the culture space 220 and the other is installed in the culture solution chamber 230, and in this case, an inner surface of the second partition wall 212a forming an outer wall of the culture space 220 is extended inward by a corresponding length, and an electrode guide hole 224 is formed in the extended portion to guide the trans-epithelial electrical resistance measuring electrode 250 to the interior of the culture space 220.

The trans-epithelial electrical resistance measuring electrode 250 installed in the culture solution chamber 230 is guided by the electrode guide hole 224 formed adjacent to the culture solution inlet/outlet hole 213 formed on an upper side of the second partition wall 212a and enters the culture solution chamber 230.

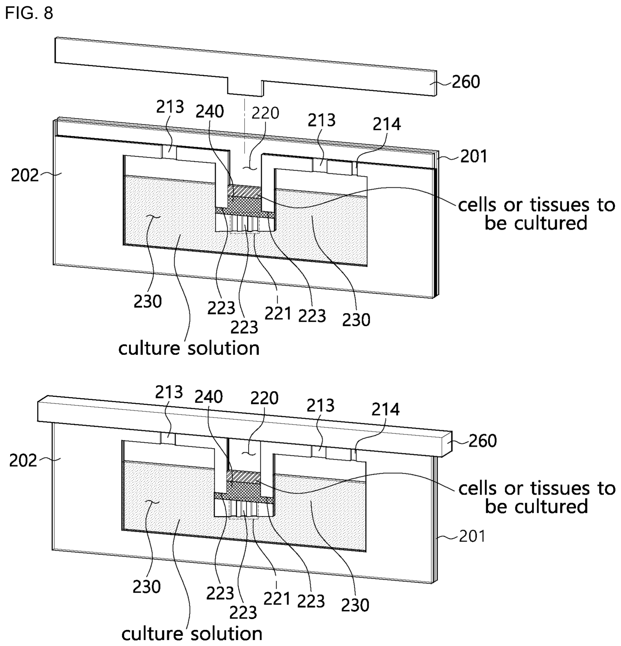

Also, referring to FIG. 8, the upright culture slide device for tissue or cell culture according to the second embodiment of the present invention includes a cover 260, and the cover 260 is coupled to upper sides of the pair of slides 201 and 202 facing each other to seal the culture space 220 of the spacer member 210.

In this case, the cover 260 may be provided in a form that is inserted between the pair of slides 201 and 202 and a form that wraps upper sides of the pair of slides 201 and 202.

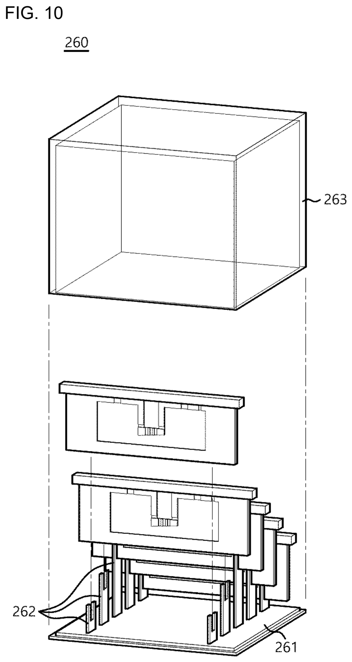

In addition, referring to FIG. 10, the upright culture slide device for tissue or cell culture according to the second embodiment of the present invention includes a culture box 260, and the culture box 260 accommodates a plurality of upright culture plate devices therein, and the culture box 260 forms a hexahedron having a space therein.

In this case, the culture box 260 includes a mounting stand 261 and a box cover 263, and the mounting stand 261 has a plurality of mounting members 262 arranged in pairs at regular intervals on an upper surface of a plate.

Here, heights of the mounting members are different from each other, and accordingly, upright culture slide devices for tissue or cell culture may be mounted in multiple stages.

In addition, the box cover 263 is placed on an upper side of the mounting stand 261, and the box cover 263 is placed on the upper side of the mounting stand 261 to solve a problem of evaporation of a culture solution during a culturing process.

Various examples using the upright culture slide device for tissue or cell culture according to the present invention are as follows.

### [Example 1]

Hydrogel including collagen extracellular matrix was formed in an H-shaped cell-accommodating gasket among structures described above, and adult hair was placed therein and cultured.

A root tissue structure of a corresponding hair continued culturing while a culture solution was supplied.

### [Example 2]

An upright culture slide device for tissue or cell culture according to the second embodiment of the present invention was manufactured to perform an experiment (in vitro) on the formation of artificial skin tissue through co-culture of epidermal and dermal cells, and an experiment (ex vivo) on tissue culture was performed by extracting skin tissue from an adult mouse and placing the skin tissues on a culture slide.

Both cultured tissue survived for a period of 2 to 4 weeks while a culture solution was supplied, and the cells maintained a skin tissue-specific layered structure.

### [Example 3]

By using a trans-epithelial electrical resistance electrode proposed by the present invention, a TEER value on a culture slide on which tissue was cultured and a TEER value on a culture slide accommodating only collagen hydrogel were measured and compared with each other.

A difference between resistance measurement values of two culture slides in a state where the tissue was alive was checked.

### [Example 4]

As a result of culturing by mounting a culture slide on a culture box or so on illustrated in FIG. 10, culturing was possible for 2 to 4 weeks while minimizing moisture evaporation of a culture solution.

The culture solution was replaced by using a syringe to remove waste and supply nutrients/signal materials, and tissue culture was successful without bacterial contamination during a culturing period.

The present invention is described with reference to the embodiments illustrated in the drawings, but these are merely examples, and those skilled in the art will understand that various modifications and equivalent other embodiments may be derived therefrom. Therefore, the true technical protection scope of the present invention should be determined by the technical idea of the attached patent claims.

## Claims

1. An upright culture slide device for tissue or cell culture comprising:
a pair of slides (101,102) having opposing surfaces facing each other and arranged to be separated from each other;
a culture solution-accommodating gasket (110) provided between the pair of slides (101, 102) and having an open upper portion to form a culture solution chamber (130) for accommodating a culture solution;
a cell-accommodating gasket (120) provided inside the culture solution-accommodating gasket (110), including a wall of a culture space in which cells or tissues are cultured, having an open upper portion, and including a support that supports the culture space (140); and
a semipermeable membrane support layer (141) formed in the cell-accommodating gasket (120) and allowing a culture solution accommodated in the culture solution-accommodating gasket (110) to flow into the cell-accommodating gasket (120).

2. The upright culture slide device for tissue or cell culture of claim 1, wherein the cell-accommodating gasket (120) includes a horizontal bar (121) having a length in a horizontal direction, and vertical bars (122) formed respectively on left and right sides of the horizontal bar (121) to have lengths in a vertical direction and having upper sides forming the wall of the culture space (140), and having lower sides forming the support.

3. The upright culture slide device for tissue or cell culture of claim 2, wherein the lower sides of the vertical bars (122) of the cell-accommodating gasket (120) which form the horizontal bar (121) and the support have rough surfaces such that the culture solution may move along the rough surfaces.

4. The upright culture slide device for tissue or cell culture of claim 1, further comprising:
a cover (150) coupled to upper sides of the pair of slides (101,102) facing each other and sealing upper portions of the culture space (140) and the culture solution chamber (130) from the outside; and
a stand (160) coupled to lower sides of the pair of slides facing each other and causing the pair of slides (101,102) to upright in a vertical direction.

5. The upright culture slide device for tissue or cell culture of claim 1, wherein a plurality of culture solution-accommodating gaskets (110) and a plurality of cell solution-accommodating gaskets (120) are provided between the pair of slides (101,102) facing each other to form a plurality of culture solution chambers (130) and the culture space (140).

6. An upright culture slide device for tissue or cell culture comprising:
a pair of slides (201,202) having opposing surfaces facing each other with a corresponding interval; and
a spacer member (210) provided between the pair of slides (201,202), forming a culture space (220), in which cells or tissues are cultured, in a center of the spacer member (210), and forming a culture solution chamber (230), in which a culture solution is accommodated, around the culture space (220).

7. The upright culture slide device for tissue or cell culture of claim 6, wherein the spacer member (210) forms a space having a corresponding area, and includes a culture solution chamber and a culture space partitioned by a partition wall provided in the space and forming an outer wall of the culture space (220).

8. The upright culture slide device for tissue or cell culture of claim 7, wherein an upper side of the partition wall of the spacer member (210) has a culture solution inlet/outlet hole (213) through which a culture solution is injected into the culture solution chamber (230) from an outside or the culture solution accommodated in the culture solution chamber (230) is discharged to the outside, and an air inlet/outlet hole (214) through which air in the culture solution chamber (230) flows to the outside when the culture solution is injected into the culture solution chamber (230).

9. The upright culture slide device for tissue or cell culture of claim 7, wherein a microchannel (221), through which the culture solution is supplied to the culture space (220) to the culture chamber due to a capillary phenomenon, is formed on a lower side of the partition wall forming the outer wall of the culture space (220).

10. The upright culture slide device for tissue or cell culture of claim 7, wherein a bottom surface of the culture space (220) formed by the partition wall is formed as an inclined surface (222) corresponding to a flow direction of the culture solution when the culture solution is injected into the culture solution chamber (230).

11. The upright culture slide device for tissue or cell culture of claim 7, wherein a semipermeable membrane support layer (240) is formed on a lower side of the culture space (220) to support cells or tissues cultured in the culture space (220).

12. The upright culture slide device for tissue or cell culture of claim 11, wherein a fixed hole formed by extending a part of a lower side of the semipermeable membrane support layer (240) is provided on a lower side of the partition wall forming the outer wall of the culture space (220).

13. The upright culture slide device for tissue or cell culture of claim 7, further comprising:
a pair of trans-epithelial electrical resistance (TEER) measuring electrodes (250), one of which is installed in the culture space (220) and the other of which is installed in the culture solution chamber (230), measuring electrical resistance between the culture space and the culture solution chamber (230).

14. The upright culture slide device for tissue or cell culture of claim 13, wherein the trans-epithelial electrical resistance measuring electrode (250) enters the culture space (220) and the culture solution chamber (230) by being guided by an electrode guide hole (224) formed in an extended portion that is formed by extending an inner surface of the partition wall forming the outer wall of the culture space by a corresponding length, and by an electrode guide hole (224) formed adjacent to the culture solution inlet/outlet hole (213) formed on the upper side of the partition wall.

15. The upright culture slide device for tissue or cell culture of claim 7, wherein the spacer member (210) includes a first spacer pad (211) forming a space having a corresponding area therein and including a culture solution chamber (230) and a culture space (220) formed in the space and partitioned by a first partition wall (221a) forming the outer wall of the culture space (220), and a second spacer pad (212) forming a space having a corresponding area therein and including a culture solution chamber (230) and a culture space (220) formed in the space and partitioned by a second wall forming the outer wall of the culture space (220), and
the first spacer pad (211) is coupled to the second spacer pad (212).
